**Europäisches Patentamt**

**European Patent Office**

**Office européen des brevets**

(19)

(11) Veröffentlichungsnummer : **0 060 551**
**B1**

(12) **EUROPÄISCHE PATENTSCHRIFT**

(45) Veröffentlichungstag der Patentschrift :
25.07.84

(21) Anmeldenummer : 82102098.9

(22) Anmeldetag : 15.03.82

(51) Int. Cl.³ : **C 07 D213/10**, C 07 D213/09,
C 07 D213/12

(54) Verfahren zur Herstellung von 3-Picolin.

(30) Priorität : 18.03.81 CH 1836/81

(43) Veröffentlichungstag der Anmeldung :
22.09.82 Patentblatt 82/38

(45) Bekanntmachung des Hinweises auf die Patenterteilung : 25.07.84 Patentblatt 84/30

(84) Benannte Vertragsstaaten :
AT BE CH DE FR GB IT LI LU NL SE

(56) Entgegenhaltungen :
DE-A- 1 695 296
DE-A- 1 965 010
DE-A- 2 203 384
DE-A- 2 703 070

(73) Patentinhaber : **LONZA AG**
**Gampel/Wallis (CH)**

(72) Erfinder : **Dinkel, Rolf, Dr.**
**Lärchenstrasse 8**
**Münchenstein Kanton Baselland (CH)**

(74) Vertreter : **von Füner, Alexander, Dr. et al**
**Patentanwälte v. Füner, Ebbinghaus, Finck Mariahilf-**
**platz 2 & 3**
**D-8000 München 90 (DE)**

**0 060 551**

**Beschreibung**

Die vorliegende Erfindung betrifft ein Verfahren zur Herstellung von 3-Picolin.

Pyridinbasen stellen wichtige Zwischenprodukte in der chemischen Industrie dar, so z. B. bei der Herstellung von Nicotinsäure oder Nicotinsäureamid. Es sind verschiedene Verfahren zur Herstellung von Pyridinbasen bekannt.

2-Methyl-5-äthylpyridin wird heute großtechnisch im Flüssigphasenverfahren aus Acetaldehyd oder Paraldehyd und Ammoniak in Gegenwart verschiedenster Katalysatoren, wie z. B. Ammoniumsalze, hergestellt. Als Nebenprodukt fallen kleine Mengen an 2- und 4-Picolin an.

2- und 4-Picolin werden heute in Gasphasenreaktionen bei Temperaturen von ca. 400 °C aus Acetaldehyd und Ammoniak unter Verwendung von Festbett- oder Fließbettkatalysatoren auf Basis von Aluminiumsilikat hergestellt.

Für die Erzeugung von Pyridin sowie von 3-Picolin, welches immer größere Bedeutung bekommt, werden heute Gasphasenreaktionen angewendet, wobei durch Zugabe von Formaldehyd zum Acetaldehyd die Bildung von 2- und 4-Picolin zu Gunsten von 3-Picolin unterdrückt wird. Auch diese Umsetzungen finden im Festbett oder Fließbett mit Aluminiumsilikat als Katalysator bei Temperaturen von etwa 400 °C statt. Nach diesen Verfahren werden Ausbeuten an 3-Picolin in der Größenordnung von höchsten 40 bis 44 % erzielt. Daneben fallen große Mengen an Pyridin an.

Es ist auch bekannt, daß anstelle von gesättigten Aldehyden von ungesättigten Aldehyden, wie z. B. Acrolein oder Crotonaldehyd, ausgegangen werden kann. Diese Reaktionen finden in der Gasphase bei hohen Temperaturen statt ; die Ausbeuten liegen im wesentlichen gleich hoch wie bei der Verwendung von gesättigten Aldehyden als Ausgangsmaterial.

Ziel der vorliegenden Erfindung ist es, 3-Picolin in hohen Ausbeuten herzustellen, wobei die Bildung von Pyridin möglichst unterdrückt werden soll.

Erfindungsgemäß wird dies dadurch erreicht, daß man Acetaldehyd bzw. dessen Polymere und/oder Acetaldehydacetale und/oder Crotonaldehyd mit Formaldehyd bzw. dessen Polymere und/oder Formaldehydacetal und/oder Hexamethylentetramin bei einem Molverhältnis von Acetaldehyd bzw. dessen Polymere und/oder Acetaldehydacetalen zu Formaldehyd bzw. dessen Polymere und/oder Formaldehydacetale von 1 : 0,5 bis 1,2 bzw. Crotonaldehyd zu Formaldehyd bzw. dessen Polymere und/oder Formaldehydacetalen von 1 : 1 bis 1 : 2,4 bzw. Acetaldehyd bzw. dessen Polymere und/oder Acetaldehydacetalen zu Hexamethylentetramin von 1 : 0,083 bis 1 : 0,2 in flüssiger wässeriger Phase bei Temperaturen von 180 bis 280 °C im geschlossenen Gefäß in Gegenwart von Amiden von Carbonsäuren in einer Menge von 0,1 bis 8 Mol. bezogen auf die Molsumme der oben genannten Verbindungen, umsetzt.

Unter Acetaldehyd im Sinne der Erfindung sind auch dessen Polymere, wie z. B. Paraldehyd, zu verstehen ; unter Formaldehyd auch dessen Polymere, wie z. B. Trioxan.

Vorzugsweise gelangen die einzelnen möglichen Komponenten jeweils nur einzeln zum Einsatz. So Acetaldehyd oder ein Acetaldehydacetal oder Crotonaldehyd (Edukt 1) und Formaldehyd oder ein Formaldehydacetal oder Hexamethylentetramin (Edukt 2).

Die für die Reaktion wichtigen Amide von Carbonsäuren können solche von aliphatischen, von aromatischen oder von heterocyclischen Mono- oder Polycarbonsäuren sein. Es handelt sich also beispielsweise um Amide der Kohlensäure (Harnstoff), der Essigsäure, der Propionsäure, der Buttersäure, der Bernsteinsäure, der Glutarsäure, der Adipinsäure, der Benzoesäure, der Phthalsäure, der Terephthalsäure, der Pyridincarbonsäuren wie Nicotinsäure oder Isonicotinsäure.

Die Amide der Carbonsäuren werden in Mengen von 0,1 bis 8 Mol, bezogen auf die Mol-Summe der Edukte, angewendet.

Für die Bildung von 3-Picolin aus Acetaldehyd und/oder Acetaldehydacetalen und/oder Crotonaldehyd mit Formaldehyd und/oder Formaldehydacetalen und/oder Hexamethylentetramin ist es vorteilhaft, die Umsetzung in Gegenwart von Ammoniak durchzuführen. Wird Ammoniak angewendet, so kann er entweder gasförmig oder als wässerige Lösung eingesetzt werden.

Werden miteinander nicht mischbare flüssige Ausgangsmaterialien, wie z. B. Paraldehyd zusammen mit wässrigem Formaldehyd, verwendet, so ist es vorteilhaft, zur Homogenisierung kleine Mengen Homogenisierungsmittel, wie Alkohole, cyclische Aether, vorzugsweise jedoch vorgebildetes 3-Picolin, einzusetzen, oder die nicht mischbaren flüssigen Ausgangsmaterialien mit je einer eigenen Pumpe in den Reaktor einzuspeisen.

Nach dem Verfahren der Erfindung wird überraschenderweise 3-Picolin in guten Ausbeuten erhalten und die Bildung von Pyridin stark unterdrückt. Als Nebenprodukte fallen 3-Aethylpyridin sowie kleine Mengen an 2,5-Dimethylpyridin, 3,5-Dimethylpyridin und 2-Methyl-5-äthylpyridin an.

Das Verfahren der Erfindung wird mit einem Molverhältnis Acetaldehyd und/oder Acetaldehydacetale zu Formaldehyd und/oder Formaldehydacetale von 1 zu 0,5 bis 1,2, vorzugsweise von 1 zu 0,8 bis 1 zu 1, durchgeführt.

Wird Crotonaldehyd anstelle von Acetaldehyd und/oder Acetaldehydacetalen verwendet, so verschiebt sich das Molverhältnis Crotonaldehyd zu Formaldehyd und/oder Formaldehydacetalen entsprechend auf 1 zu 1 bis 1 zu 2,4.

2

Wird Hexamethylentetramin anstelle von Formaldehyd und/oder Formaldehydacetalen verwendet, so verschiebt sich das Molverhältnis Acetaldehyd und/oder Acetaldehydacetale zu Hexamethylentetramin entsprechend auf 1 zu 0,083 bis 1 zu 0,2.

Die Reaktionstemperaturen liegen, bei 180 bis 280 °C, zweckmässig bei 205 bis 240 °C, vorzugsweise bei 225 bis 235 °C.

Die Reaktion wird in flüssiger Phase (wässriger Phase) unter einem Druck, der sich bei der Reaktion im geschlossenen Gefäss bei vorgegebener Temperatur einstellt, durchgeführt. Es ist vorteilhaft, während der Reaktion den Reaktionsansatz zu rühren.

Die Menge an Ammoniak liegt bei 0,5 bis 3 Mol Ammoniak pro Mol Edukt, zweckmässig bei 0,6 bis 1,0 Mol pro Mol Edukt.

Die Zugabe des Aldehyds erfolgt zweckmässig nach Massgabe seines Verbrauches. So ist es beispielsweise günstig, beim Arbeiten in einem 2-Liter-Behälter und bei Einsatz von 350 ml Aldehyd diesen kontinuierlich während 30 bis 90 Minuten zuzusetzen. Bei anderen Bedingungen sind die entsprechenden Zugabezeiten zu wählen.

Am Ende der gewünschten Reaktionsperiode wird die Temperatur auf etwa Raumtemperatur gesenkt und das 3-Picolin auf bekannte Weise aus der Reaktionsmischung gewonnen. Eine Methode besteht darin, dass man den pH-Wert der Wasserphase zuerst in den basischen Bereich bringt und dann das organische Material aus der wässrigen Reaktionsmischung mit einem organischen Lösungsmittel, z. B. Benzol, Toluol, Xylol, Methylenchlorid, Chloroform, Aether und dergleichen, extrahiert. Das organische Lösungsmittel wird dann abgedampft und man erhält 3-Picolin durch fraktionierte Destillation. Im Rahmen der Erfindung können auch beliebige andere Methoden zur Abtrennung und Gewinnung des Produktes angewendet werden.

Obgleich die Erfindung als diskontinuierliches Verfahren beschrieben worden ist, kann das Verfahren auch im Rahmen der vorliegenden Erfindung kontinuierlich betrieben werden. Bei einer Ausführungsform eines kontinuierlichen Verfahrens werden die Reaktionsteilnehmer kontinuierlich in einen geeigneten Druckreaktor eingeführt, aus dem die Reaktionsmischung kontinuierlich abgezogen wird. Die Reaktionsprodukte werden daraus abgetrennt, die wässrige Phase aufkonzentriert und unveränderte Reaktionsteilnehmer werden dann wieder ergänzt und in das Reaktionsgefäss zurückgeführt.

Das kontinuierliche Verfahren kann in jedem Reaktor durchgeführt werden, der eine innige Vermischung der Reaktionsteilnehmer unter heftigem Rühren gestattet, z. B. in einem kontinuierlich gerühten Tankreaktor.

## Beispiel 1

1 140 ml einer wässrigen Lösung von 134,7 g Acetamid und 50,1 g Ammoniak (pH der Lösung = 11,9) wurden in einem 2-Liter-Autoklaven auf 230 °C erhitzt und bei 1 500 UpM gerührt. In diese Lösung wurde innert 64 Minuten kontinuierlich ein Gemisch aus 117,6 g Acetaldehyd und 213,3 g einer 30,2 %-igen wässrigen Formaldehydlösung eingepumpt (Molverhältnis = 1 zu 0,80). Dabei variierte der Reaktionsdruck zwischen 34 und 32 bar. Nach beendeter Zugabe des Aldehydgemisches wurde die Reaktionsmasse während 10 Minuten bei 230 °C weitergerührt und dann auf Raumtemperatur abgekühlt. Schliesslich erfolgte eine Extraktion mit 3 × 100 ml Methylenchlorid sowie eine gaschromatographische Analyse der vereinigten Methylenchloridextrakte, wobei sich folgende Produkte mit den auf eingesetzten Acetaldehyd (A) bzw. Formaldehyd (F) bezogenen Ausbeuten ergaben :

Pyridin 1,3 % (A) ; 3-Picolin 57,7 % (F) ; 3-Aethylpyridin 15,8 % (A) ; 2,5-Lutidin 5,2 % (A) ; 3,5-Lutidin 0,7 % (F) ; 2-Methyl-5-äthylpyridin 1,6 % (A).

Sämtliche gaschromatographischen Analysen wurden unter Verwendung eines internen Standards sowie unter Berücksichtigung von Flächenkorrekturfaktoren durchgeführt.

## Beispiele 2-7

Gemäss nachstehenden Tabellen

(Siehe Tabelle I, Seite 4 f.)

Tabelle I

| Bei-spiel | Säureamid | Säure-amid g | NH$_3$ g | pH | CH$_3$CHO g | CH$_2$O-Lösung g | CH$_2$O-Gehalt % | Dosier-dauer Min. | Druck bar |
|---|---|---|---|---|---|---|---|---|---|
| | **Eduktmenge und Reaktionsbedingungen** | | | | | | | | |
| 2 | CH$_3$CH$_2$CH$_2$CONH$_2$ | 87,1 | 85,1 | 12,0 | 117,6 | 213,3 | 30,6 | 61 | 41-39 |
| 3 | (CH$_2$CONH$_2$)$_2$ | 19,0 | 85,1 | 10,9 | 117,4 | 213,3 | 30,4 | 62 | 42-40 |
| 4 | PhCONH$_2$ | 30,7 | 85,1 | 11,3 | 117,4 | 213,3 | 30,2 | 60 | 38-37 |
| 5 | (Pyridin)—CONH$_2$ | 122,1 | 85,1 | 11,4 | 117,7 | 213,3 | 30,5 | 62 | 38-36 |
| 6 | NH$_2$CONH$_2$ | 60,1 | 85,1 | 12,5 | 117,4 | 213,3 | 30,6 | 59 | 48-54 |
| 7 | CH$_3$CONH$_2$ | 229,2 | ∅ | 5,3 | 117,7 | 213,3 | 30,2 | 66 | 26-28 |

Tabelle II

| Bei-spiel | (A) | (F) | (A) | (A) | (F) | (A) |
|---|---|---|---|---|---|---|
| | **Ausbeuten %** | | | | | |
| 2 | 1,5 | 55,4 | 14,7 | 5,4 | 1,0 | 1,5 |
| 3 | 2,1 | 49,8 | 12,0 | 5,5 | 1,4 | 1,4 |
| 4 | 1,9 | 47,3 | 11,5 | 5,1 | 1,3 | 1,3 |
| 5 | 1,8 | 55,9 | 13,8 | 5,2 | 0,9 | 1,5 |
| 6 | 1,7 | 52,1 | 14,4 | 4,7 | 1,4 | 1,4 |
| 7 | 1,3 | 52,7 | 15,2 | 4,5 | 0,8 | 2,0 |

**Ansprüche**

1. Verfahren zur Herstellung von 3-Picolin, dadurch gekennzeichnet, daß man Acetaldehyd bzw. dessen Polymere und/oder Acetaldehydacetale und/oder Crotonaldehyd mit Formaldehyd bzw. dessen Polymere und/oder Formaldehydacetal und/oder Hexamethylentetramin bei einem Molverhältnis von Acetaldehyd bzw. dessen Polymere und/oder Acetaldehydacetalen zu Formaldehyd bzw. dessen polymere und/oder Formaldehydacetale von 1 : 0,5 bis 1,2 bzw. Crotonaldehyd zu Formaldehyd bzw. dessen Polymere und/oder Formaldehydacetalen von 1 : 1 bis 1 : 2,4 bzw. Acetaldehyd bzw. dessen Polymere und/oder Acetaldehydacetalen zu Hexamethylentetramin von 1 : 0,083 bis 1 : 0,2 in flüssiger wässeriger Phase bei Temperaturen von 180 bis 280 °C im geschlossenen Gefäß in Gegenwart von Amiden von Carbonsäuren in einer Menge von 0,1 bis 8 Mol, bezogen auf die Molsumme der oben genannten Verbindungen, umsetzt.

2. Verfahren nach Anspruch 1, dadurch gekennzeichnet, daß man die Umsetzung in Gegenwart von Ammoniak in einer Menge von 0,5 bis 3 Mol Ammoniak/Mol der Ausgangsprodukte durchführt.

3. Verfahren nach einem der Ansprüche 1 oder 2, dadurch gekennzeichnet, daß man bei 205 bis 240 °C arbeitet.

## Claims

1. Process for the preparation of 3-picoline, characterised in that one reacts acetaldehyde or its polymers and/or acetaldehyde acetals and/or crotonaldehyde with formaldehyde or its polymers and/or formaldehyde acetal and/or hexamethylenetetramine in a mole ratio of acetaldehyde or its polymers and/or acetaldehyde acetals to formaldehyde or its polymers and/or formaldehyde acetals of 1 : 0,5 to 1.2 or of crotonaldehyde to formaldehyde or its polymers and/or formaldehyde acetals of 1 : 1 to 1 : 2.4 or of acetaldehyde or its polymers and/or acetaldehyde acetals to hexamethylenetetramine of 1 : 0,083 to 1 : 0.2 in liquid aqueous phase at temperatures of 180 to 280 °C. in a closed vessel in the presence of amides of carboxylic acids in an amount of 0.1 to 8 mole, referred to the mole sum of the above-mentioned compounds.

2. Process according to claim 1, characterised in that one carries out the reaction in the presence of ammonia in an amount of 0.5 to 3 mole ammonia/mole of the starting products.

3. Process according to one of claims 1 or 2, characterised in that one operates at 205 to 240 °C.

## Revendications

1. Procédé pour la préparation de 3-picoline, caractérisé en ce que l'on fait réagir de l'acétaldéhyde ou ses polymères et/ou des acétals d'acétaldéhyde et/ou de l'aldéhyde crotonique avec du formaldéhyde ou ses polymères et/ou des acétals de formaldéhyde et/ou de l'hexaméthylènetétramine à un rapport molaire de l'acétaldéhyde ou de ses polymères et/ou des acétals d'acétaldéhyde au formaldéhyde ou à ses polymères et/ou aux acétals de formaldéhyde de 1 : 0,5 à 1,2, ou de l'aldéhyde crotonique au formaldéhyde ou à ses polymères et/ou aux acétals de formaldéhyde de 1 : 1 à 1 : 2,4, ou de l'acétaldéhyde ou de ses polymères et/ou des acétals d'acétaldéhyde à l'hexaméthylènetétramine de 1 : 0,083 à 1 : 0,2 en phase aqueuse liquide à des températures de 180 à 280 °C en récipient fermé en présence d'amides d'acides carboxyliques en une quantité de 0,1 à 8 molaire par rapport à la somme molaire des composés mentionnés plus haut.

2. Procédé selon la revendication 1, caractérisé en ce qu'on effectue la réaction en présence d'ammoniac en une quantité de 0,5 à 3 moles d'ammoniac/mole des produits de départ.

3. Procédé selon l'une des revendications 1 ou 2, caractérisé en ce qu'on travaille à 205 à 240 °C.